# EUROPÄISCHE PATENTANMELDUNG

(11) **EP 1 790 330 A2**
(43) Veröffentlichungstag der Anmeldung: **30.05.2007**
(21) Anmeldenummer: 06020514.3
(22) Anmeldetag: 29.09.2006
(51) Int. Cl.: A61K 8/97, A61K 8/64, A61Q 19/08, A61Q 17/04

(54) **Kosmetische und dermatologische Zusammensetzungen mit Oligopeptiden und Apfelextrakt**

(30) Priorität: 14.10.2005 DE 102005049679; 27.09.2006 DE 102006046076
(71) Anmelder: Henkel Kommanditgesellschaft auf Aktien, 40589 Düsseldorf (DE)
(72) Erfinder: Holtkötter, Olaf, 50354 Hürth (DE); Waldmann-Laue, Marianne, 40789 Monheim (DE); Döring, Thomas, 41542 Dormagen (DE)

(57) **Zusammenfassung**

Die Erfindung betrifft topische kosmetische oder dermatologische Zusammensetzungen, die in einem geeigneten kosmetischen oder dermatologischen Träger mindestens ein ausgewähltes Oligopeptid und mindestens einen Apfelextrakt, insbesondere Apfelkern- oder Apfelfruchtextrakt, enthalten.

## Beschreibung

Die Erfindung betrifft topische kosmetische oder dermatologische Zusammensetzungen, die in einem geeigneten kosmetischen oder dermatologischen Träger mindestens ein ausgewähltes Oligopeptid und mindestens einen Apfelextrakt, insbesondere Apfelkern- oder Apfelfruchtextrakt, enthalten.

Die Haut des Menschen altert während seiner Lebenszeit stetig einerseits durch extrinsische Faktoren, wie Licht oder Wind, oder durch intrinsische Alterung. Das Schönheitsideal ist heutzutage davon geprägt, eine faltenfreie Haut, insbesondere Gesichtshaut, zu besitzen, die dazu noch glatt, straff und elastisch ist.

Es wurde festgestellt, dass die bekannten Zusammensetzungen bestimmte Alterungserscheinungen der Haut, insbesondere die lichtinduzierten Hautalterungserscheinungen, wie Fältchen, nicht immer in zufriedenstellender Weise verbessern können.

In zahlreichen unabhängigen *in vivo* und *in vitro*-Studien wurde umfassend beschrieben, dass die All-trans-Retinsäure sowie deren Isoformen in der Lage sind, Alterserscheinungen der Haut zu reduzieren. Zu den durch die Retinsäure hervorgerufenen besonders erwünschten makroskopischen Hautveränderungen zählen u.a. die Reduktion der Falten und Hautrauheit, eine leichte Verminderung der Farbintensität von Altersflecken und Sommersprossen und eine Verbesserung der Dermatoheliose. Auf der mikroskopischen Seite äußern sich diese Veränderungen u.a. durch eine Verdickung der Epidermis und eine gesteigerte Kollagenneusynthese.

Nachteilig ist, dass die Anwendung von Retinsäure auf der Haut häufig zu unerwünschten Nebenreaktionen, wie beispielsweise einer deutlichen Hautirritation oder Ödembildung, führt.

Aufgabe der vorliegenden Erfindung war es daher, eine kosmetische oder dermatologische topische Zusammensetzung bereitzustellen, die eine verbesserte Behandlung von Fältchen, insbesondere von Fältchen, die auf die lichtinduzierte Hautalterung zurückzuführen sind, ermöglicht und dabei bei den Zellen der Haut ein ähnliches Expressionsprofil wie die Retinsäure hervorruft.

Eine weitere Aufgabe der vorliegenden Erfindung war es, eine kosmetische oder dermatologische topische Zusammensetzung bereitzustellen, die das Erscheinungsbild der Haut, insbesondere der lichtgealterten Haut, verbessert.

Eine weitere Aufgabe der vorliegenden Erfindung war es, eine kosmetische oder dermatologische topische Zusammensetzung mit verringertem Allergiepotenzial bereitzustellen, die das Erscheinungsbild der Haut, insbesondere der lichtgealterten Haut, verbessert.

Eine weitere Aufgabe der vorliegenden Erfindung war es, eine kosmetische oder dermatologische topische Zusammensetzung auf der Basis ethisch akzeptierter Wirkstoffe bereitzustellen, die das Erscheinungsbild der Haut, insbesondere der lichtgealterten Haut, verbessert.

Überraschend wurde gefunden, dass kosmetische oder dermatologische topische Zusammensetzungen, die mindestens ein Oligopeptid, das einen N-C₂-C₂₄-Acylrest aufweisen und/oder verestert sein kann, und mindestens einen Apfelextrakt, insbesondere Apfelkern- oder Apfelfruchtextrakt, enthalten, in für den Fachmann nicht vorhersehbarer Weise die Nachteile des Standes der Technik beseitigen. Weiterhin wurde überraschend gefunden, dass derartige Wirkstoffkombinationen das Genprofil von Hautzellen in ähnlicher Weise wie Retinsäure beeinflussen.

Gegenstand der vorliegenden Erfindung sind kosmetische oder dermatologische topische Zusammensetzungen, die in einem geeigneten kosmetischen oder dermatologischen Träger mindestens ein Oligopeptid, das mindestens einen N-C₂-C₂₄-Acylrest aufweisen und/oder verestert sein kann, und mindestens einen Apfelextrakt, insbesondere Apfelkern- oder Apfelfruchtextrakt, enthalten.

Ein besonders bevorzugter Apfelextrakt ist Apfelkernextrakt. Besonders bevorzugte Hndelsprodukte, die Apfelkernextrakt enthalten, stammen aus der Serie "Ederline" der Firma Vincience. Ederline enthält Phytohormone, Isoflavonoide, Phytosterole, Triterpenoide, Tocopherole und natürliche Wachse.
Das Produkt Ederline ist einmal in wasserlöslicher Form als Ederline-H (INCl: PEG-40 Hydrogenated Castor Oil, PPG-2-Ceteareth-9, Pyrus Malus (Apple) Fruit Extract), zum anderen in öllöslicher Form als Ederline-L (INCl: Hexyldecanol, Pyrus Malus (Apple) Fruit Extract) erhältlich. Der erfindungsgemäß besonders bevorzugte Rohstoff Ederline L weist einen Aktivsubstanzgehalt an reinem Apfelkernextrakt von 3 Gew.-% auf.
Erfindungsgemäß bevorzugte Mengen an Ederline sind 0,1-10 Gew.-%, besonders bevorzugt 1 - 5 Gew.-% und außerordentlich bevorzugt 1,5 - 3 Gew.-%, jeweils bezogen auf die gesamte erfindungsgemäße Zusammensetzung.
Bezogen auf den Gehalt an aktiven Wirkstoffen, wird der Apfelextrakt, insbesondere der Apfelkernextrakt oder Apfelfruchtextrakt, erfindungsgemäß bevorzugt in einer Gesamtmenge von 0,001 - 1 Gew.-%, besonders bevorzugt 0,01 - 0,5 Gew.-% und außerordentlich bevorzugt 0,03 - 0,1 Gew.-%, jeweils bezogen auf die gesamte erfindungsgemäße Zusammensetzung, eingesetzt.

Unter den in den erfindungsgemäßen Zusammensetzungen enthaltenen Oligopeptiden, die man auch als Aminosäureoligomere bezeichnen kann, werden erfindungsgemäß Peptide mit 2 - 30, bevorzugt 2-15, besonders bevorzugt 3, 4, 5 oder 6, Aminosäureeinheiten, verstanden.
Die Aminosäuren und/oder die N-C₂-C₂₄-Acylaminosäuren sind erfindungsgemäß ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Histidin, Glycin, Prolin, Arginin, Valin und Glutamin.

Der C₂-C₂₄-Acylrest, mit dem die genannten Aminosäuren an der Aminogruppe derivatisiert sein können, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Besonders bevorzugt ist die Derivatisierung mit einem Palmitoyl-, Stearoyl-, Elaidoyl-, Arachidoyl-, Behenoyl oder Cocoyl-Rest, außerordentlich bevorzugt ist der Palmitoyl-Rest.

Mit den vorgenannten C₂-C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift PDHP von der Firma Seppic erhältlich ist.

Die physiologisch verträglichen Salze der erfindungsgemäß bevorzugten Oligopeptide, die Säuregruppen enthalten und über diese Säuregruppen Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Ebenfalls bevorzugt sind Salze der erfindungsgemäß bevorzugten Oligopeptide, die über die Aminogruppen bzw. Ammoniumgruppen gebildet werden, das heißt, solche Salze, in denen der Oligopeptid-Anteil als Kation vorliegt. Besonders bevorzugte Beispiele für anionische Gegenionen sind Chlorid und Trifluoracetat.

Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere oder Oligopeptide stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z. B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere oder Oligopeptide können als Wirkstoffe gegen die Hautalterung verwendet werden.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCl-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, N-Palmitoyl-Lys-Val-Lys, z. B. erhältlich von der Firma Pentapharm unter der Bezeichnung SYN®-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH2.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen:
Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z. B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (IIe). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z. B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Ala-Asp-Leu-Lys-Pro-Thr (Hexapeptide-3, z. B. Peptide 02 von Vincience), Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Hexapeptide-11 (z. B. Peptamide-6 von Arch Personal Care), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), und Val-Val-Arg-Pro-Pro-Pro. Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).

Es ist erfindungsgemäß besonders bevorzugt, Kombinationen aus mindestens zwei Oligopeptiden einzusetzen. Erfindungsgemäß besonders bevorzugt sind Kombinationen aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich sind.

Gemäß einer besonders bevorzugten Ausführungsform ist der Apfelextrakt, bevorzugt der Apfelkernextrakt, mit einer Kombination aus mindestens drei Oligopeptiden einzusetzen, die sich in ihren Effekten gegenseitig verstärken und so insgesamt eine geringere Einsatzkonzentration der Oligopeptide zulassen.

Bevorzugt geeignet sind dabei Kombinationen von Apfelextrakt, bevorzugt der Apfelkernextrakt, mit drei oder mehr Oligopeptiden. Erfindungsgemäß besonders bevorzugt sind Oligopeptid-Kombinationen aus Gly-His-Lys und Gly-Gln-Pro-Arg, besonders bevorzugt die Kombination N-Palmitoyl-Gly-His-Lys und N-Palmitoyl-Gly-Gln-Pro-Arg, wie bespielsweise Matrixyl 3000 von der Firma Sederma, mit a) L-Glutamylaminoethyl-indol (z.B. Glistin von Exsymol) oder b) Lys-Val-Val, dessen N-Acylderivaten, Estern und Salzen, besonders bevorzugt N-Palmitoyl-lysyl-valyl-lysine (z. B. SynColl von der Firma Pentapharm).

Erfindungsgemäß bevorzugte Zusammensetzungen sind dadurch gekennzeichnet, dass sie mindestens ein Oligopeptid enthalten, das mindestens einen N-C₂-C₂₄-Acylrest aufweisen und/oder verestert sein kann und das ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus Gly-His-Lys und Gly-Gln-Pro-Arg, Kombinationen aus N-Palmitoyl-Gly-His-Lys und N-Palmitoyl-Gly-Gln-Pro-Arg, Kombinationen aus N-Palmitoyl-Gly-His-Lys und Gly-Gln-Pro-Arg, und Kombinationen aus Gly-His-Lys und N-Palmitoyl-Gly-Gln-Pro-Arg.

Besonders bevorzugte erfindungsgemäße Zusammensetzungen sind dadurch gekennzeichnet, dass das mindestens eine Oligopeptid in Gesamtmengen von 0,000001 - 1 Gew.-%, bevorzugt 0,00001 - 0,1 Gew.-%, besonders bevorzugt 0,0001 - 0,001 - 0,01 - 0,05 Gew.-% und außerordentlich bevorzugt 0,002 - 0,005 Gew.-%, enthalten ist.

Erfindungsgemäß bevorzugte handelsübliche Oligopeptid-Zubereitungen sind Matrixyl (ex Sederma), das einen Aktivsubstanzgehalt von 0,0127 Gew.-% aufweist, Matrixyl 3000 (ex Sederma), das einen Aktivsubstanzgehalt von mindestens 0,013 Gew.-% aufweist, Eyeliss (ex Sederma), das einen Aktivsubstanzgehalt von 0,13 Gew.-% aufweist, und SynColl (ex Pentapharm), das einen Aktivsubstanzgehalt von 0,09 - 0,11 Gew.-% aufweist, wobei sich alle Mengenangaben auf das Gewicht des Handelsproduktes tel quel beziehen.

Es wurde überraschend festgestellt, dass die besten Ergebnisse in Bezug auf Verbesserung des Hauterscheinungsbildes erzielt werden können, wenn das Gewichtsverhältnis von Apfelextrakt zu Oligopeptid(en) in einem bestimmten Bereich liegt, wobei auf jeden Fall der Apfelextrakt, insbesondere der Apfelkern- oder Apfelfruchtextrakt, im Überschuss zu dem/den Oligopeptid(en) vorliegt, wobei dieser Überschuss allerdings auch nach oben hin begrenzt sein sollte.
Weitere bevorzugte erfindungsgemäße Zusammensetzungen sind daher dadurch gekennzeichnet, dass das Gewichtsverhältnis von Apfelextrakt zur Gesamtmenge des mindestens einen Oligopeptids, das mindestens einen N-C₂-C₂₄-Acylrest aufweisen und/oder verestert sein kann, im Bereich von 15 - 250, bevorzugt 20 - 200, besonders bevorzugt 30 - 175 und außerordentlich bevorzugt 50 bis 100 bis 150 liegt, wobei sich diese Angaben auf die jeweilige Aktivsubstanz beziehen.

Überraschend wurde festgestellt, dass die hautfaltenglättende Wirkung der erfindungsgemäßen Zusammensetzungen weiter verbessert werden kann, wenn mindestens ein weiterer Wirkstoff enthalten ist, der ausgewählt ist aus Retinol, Olivenblattextrakten, mindestens einem Proteinhydrolysat aus Sojabohnen, L-Carnitin oder dessen Derivaten, insbesondere L-Carnitinestern und Acylcarnitin-Verbindungen, sowie Kombuchka (synonym Kombucha), erhältlich aus der Fermentation von gezuckertem Schwarztee mit zwei symbiotischen Mikroorganismen Saccharomyces und Xylinum.

Gemäß einer besonders bevorzugten Ausführungsform weist die Zusammensetzung zusätzlich mindestens einen weiteren Wirkstoff auf, der ausgewählt ist aus Retinol, Olivenblattextrakten, mindestens einem Proteinhydrolysat aus Sojabohnen, L-Carnitin oder dessen Derivaten, insbesondere L-Carnitinestern und Acylcarnitin-Verbindungen, sowie Kombuchka (synonym Kombucha), erhältlich aus der Fermentation von gezuckertem Schwarztee mit zwei symbiotischen Mikroorganismen Saccharomyces und Xylinum.

Kombucha oder auch Kombuchka genannt sind Produkte, die durch Fermentation von gezuckertem Schwarztee mit zwei symbiotischen Mikroorganismen Saccharomyces und Xylinum gewonnen werden und die INCI-Bezeichnung Saccharomyces/Xylinum/Black Tea Ferment tragen. Ein besonders bevorzugtes Produkt ist unter dem Handelsnamen Kombuchka von der Firma Sederma erhältlich (INCI-Bezeichnung: Saccharomyces/Xylinum/Black Tea Ferment, Glycerin, Hydroxyethylcellulose). Die Produkte sind (bei einem Wassergehalt des Produkts zwischen 70 und 90 %) erfindungsgemäß bevorzugt zu 0,0001 bis 15 Gew.-%, besonders bevorzugt 0,001 bis 10 Gew.-%, insbesondere zu 0,01 bis 7 Gew.-%, ganz besonders bevorzugt zu 0,1 bis 5 Gew.-%, außerordentlich bevorzugt zu 3 Gew.-%, in den Zusammensetzungen enthalten.

Bevorzugt wird Retinol, 3,7-Dimethyl-9-(2,6,6-trimethyl-1-cyclohexenyl)-2,4,6,8-nonatetraen-1-ol, auch bekannt als Vitamin A1, in den erfindungsgemäßen Zubereitungen eingesetzt.
Besonders bevorzugt wird Retinol mit hohen Anteilen an all-trans-Retinol in einem Isomerengemisch, insbesondere mit > 50%, bevorzugt > 75%, insbesondere > 90% all-trans-Retinol in bezug auf die Summe aller Retinolisomere eingesetzt.
Besonders geeignet ist stabilisiertes Retinol, beispielsweise von RetiStar^{®} (BASF, > 90% all-trans-Retinol), wobei insbesondere Antioxidantien wie Tocopherol und Ascorbinsäure bzw. deren Salze zur Stabilisierung hinzugefügt werden.

Retinol ist erfindungsgemäß bevorzugt zu 0,00001 bis 2 Gew.-%, besonders bevorzugt 0,00005 bis 1 Gew.-%, insbesondere zu 0,0001 bis 0,5 Gew.-%, ganz besonders bevorzugt zu 0,0005 bis 0,2 Gew.-%, beispielsweise 0,02 oder 0,025 Gew.-%, bezogen auf die Aktivsubstanz in den Zusammensetzungen enthalten.

Die erfindungsgemäß bevorzugten L-Carnitinderivate sind insbesondere ausgewählt aus Acetyl-L-Carnitin, L-Carnitin-Fumarat, L-Carnitin-Citrat, Lauroyl-L-Carnitin und besonders bevorzugt L-Carnitin-Tartrat. Die genannten L-Carnitin-Verbindungen sind beispielsweise von der Firma Lonza GmbH (Wuppertal, Deutschland) erhältlich. Besonders bevorzugt ist L-Carnitin, beispielsweise Natrulon RC-50 DG (Lonza GmbH). L-Carnitin und/oder L-Carnitinderivate sind erfindungsgemäß bevorzugt in einer Gesamtmenge von 0,001 - 10 Gew.-%, besonders bevorzugt 0,05 - 5 Gew.-%, insbesondere zu 0,01 - 3 Gew.-%, ganz besonders bevorzugt zu 0,05 - 2 Gew.-%, beispielsweise 1, 5 Gew.-%, bezogen auf das Gewicht der erfindungsgemäßen Zusammensetzungen, enthalten.

Die erfindungsgemäß bevorzugten Proteinhydrolysate aus Sojabohnen können in dem Fachmann geläufiger Weise beispielsweise durch Auspressen oder Extrahieren aus Soja sowie anschließender Hydrolyse gewonnen werden. Besonders bevorzugt sind Sojaproteinhydrolysate mit einem mittleren Molekulargewicht im Bereich von 600 - 1000 Dalton, insbesondere bevorzugt 800 Dalton. Besonders bevorzugt ist beispielsweise Ridulisse C^{®} von Silab, das einen Aktivsubstanzgehalt von 3-5 Gew.-% aufweist. Das mindestens eine Sojaproteinhydrolysat ist erfindungsgemäß bevorzugt zu 0,00001 bis 5 Gew.-%, besonders bevorzugt 0,0001 bis 3 Gew.-%, insbesondere zu 0,005 bis 1 Gew.-%, ganz besonders bevorzugt zu 0,006 bis 0,5 Gew.-%, beispielsweise 0,1 bis 0,2 Gew.-%, jeweils bezogen auf die Aktivsubstanz in der gesamten kosmetischen Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.
Erfindungsgemäß besonders bevorzugte Zusammensetzungen sind dadurch gekennzeichnet, dass sie mindestens einen Olivenblattextrakt in einer Gesamtmenge von 0,001 bis 5 Gew.-%, bevorzugt 0,01 bis 2,5 Gew.-% und besonders bevorzugt 0,05 bis 1 Gew.-%, beispielsweise 0,1 Gew.-% jeweils bezogen auf den Extrakt als Handelsprodukt tel quel in der gesamten erfindungsgemäßen Zusammensetzung, enthalten.

Ganz besonders bevorzugte Kombinationen sind Zusammensetzungen aus den Oligopeptiden Gly-His-Lys und Gly-Gln-Pro-Arg (deren N-Palmitoyl-Derivate z. B. in dem Rohstoff Matrixyl^{®} 3000 von Sederma enthalten sind) und Apfelkernextrakt (z.B. Ederline L von Vincience), jeweils zusammen mit
- L-Glutamylaminoethyl-indol (z.B. Glistin von Exsymol)
- Palmitoyl-lysyl-valyl-lysine (z.B. Syncoll von der Firma Pentapharm).
- Olivenblätterextrakt (z.B. von Frutarom)
- Kombuchka (z.B. von Sederma)
- hydrolysiertem Sojaprotein, insbesondere Sojaproteinhydrolysaten mit einem mittleren Molekulargewicht im Bereich von 600 - 1000 Dalton, insbesondere bevorzugt 800 Dalton (z.B. Ridulisse C von Silab)
- Retinol (z.B. RetiStar von BASF) oder
- L-Carnitin (z.B. Natrulon von Lonza).

Besonders bevorzugt für eine besonders effektive Anti-Falten- bzw. Anti-Aging-Leistung sind folgende Wirkstoff-Kombinationen :
N-Palmitoyl-Gly-His-Lys und N-Palmitoyl-Gly-Gln-Pro-Arg und Apfelkernextrakt (z.B. Ederline L von Vincience) und L-Carnitin;
N-Palmitoyl-Gly-His-Lys und N-Palmitoyl-Gly-Gln-Pro-Arg und Apfelkernextrakt (z.B. Ederline L von Vincience) und N-Palmitoyl-Lys-Val-Lys;
N-Palmitoyl-Gly-His-Lys und N-Palmitoyl-Gly-Gln-Pro-Arg und Apfelkernextrakt (z.B. Ederline L von Vincience) und mindestens ein Sojaproteinhydrolysat mit einem mittleren Molekulargewicht im Bereich von 600 - 1000 Dalton, insbesondere bevorzugt 800 Dalton.

Weiterhin wurde festgestellt, dass sich die kosmetische Wirkung der erfindungsgemäßen Zusammensetzungen weiter steigern lässt, wenn die erfindungsgemäßen Zusammensetzungen mindestens einen Wirkstoff, ausgewählt aus natürlichen Betainverbindungen, Vitaminen, Provitaminen und Vitaminvorstufen der Gruppen A, B, C, E, H und K und den Estern der vorgenannten Substanzen, den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, natürlich vorkommenden Xanthin-Derivaten, ausgewählt aus Coffein, Theophyllin, Theobromin und Aminophyllin, Ectoin, Kreatin, Olivenblattextrakten, Ursolsäure, Oleanol und/oder Oleanolsäure, Mono- und Polyhydroxystilbenen und deren Estern, Derivaten von methyliertem Silanol, Phytinsäure, sowie Desoxyzuckern oder Desoxyzucker-Bausteine enthaltenden Polysacchariden, enthalten.

Bevorzugte erfindungsgemäße kosmetische oder dermatologische topische Zusammensetzungen sind dadurch gekennzeichnet, dass mindestens ein Wirkstoff, ausgewählt aus natürlichen Betainverbindungen, Vitaminen, Provitaminen und Vitaminvorstufen der Gruppen A, B, C, E, H und K und den Estern der vorgenannten Substanzen, den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, natürlich vorkommenden Xanthin-Derivaten, ausgewählt aus Coffein, Theophyllin, Theobromin und Aminophyllin, Ectoin, Kreatin, Olivenblattextrakten, Ursolsäure, Oleanol und/oder Oleanolsäure, Mono- und Polyhydroxystilbenen und deren Estern, Derivaten von methyliertem Silanol, Phytinsäure, sowie Desoxyzuckern oder Desoxyzucker-Bausteine enthaltenden Polysacchariden, enthalten ist.

In einer bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Bevorzugte natürliche Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat.
Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus der Vitamingruppe A oder mindestens einen Ester hiervon in einer Gesamtmenge von 0,00001 bis 2 Gew.-%, besonders bevorzugt 0,00005 bis 1 Gew.-%, insbesondere zu 0,0001 bis 0,5 Gew.-%, ganz besonders bevorzugt zu 0,0005 bis 0,2 Gew.-%, beispielsweise 0,02 oder 0,025 Gew.-%, bezogen auf die Aktivsubstanz in der gesamten erfindungsgemäßen Zusammensetzungen enthalten.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (I) eingesetzt.

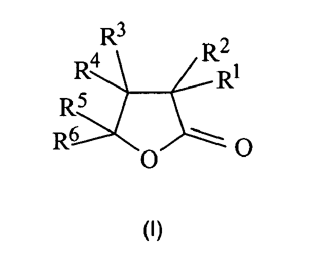

Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Erfindungsgemäß besonders bevorzugte Zusammensetzungen sind dadurch gekennzeichnet, dass sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4*S*, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇ und deren Estern und aus Pantolacton.

Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat,-succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Erfindungsgemäß besonders bevorzugte Zusammensetzungen sind dadurch gekennzeichnet, dass sie mindestens ein Vitamin K in einer Gesamtmenge von 0001 bis 1,0 Gew.-%, bevorzugt 0,05 bis 0,01 Gew.-%, besonders bevorzugt 0,1 bis 0,5 Gew.-%, jeweils bezogen auf die gesamte Zusammensetzung, enthalten.

Vitamin A-palmitat (Retinylpalmitat), Panthenol, Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen mindestens ein Flavonoid, ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.
Erfindungsgemäß besonders bevorzugte Zusammensetzungen sind dadurch gekennzeichnet, dass sie mindestens ein erfindungsgemäß bevorzugtes Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

In einer weiteren bevorzugten Ausführungsform enthalten die erfindungsgemäßen Zusammensetzungen mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.

Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Erfindungsgemäß bevorzugt sind die Ubichinone. Die erfindungsgemäß bevorzugten Ubichinone weisen die Formel (II) auf: 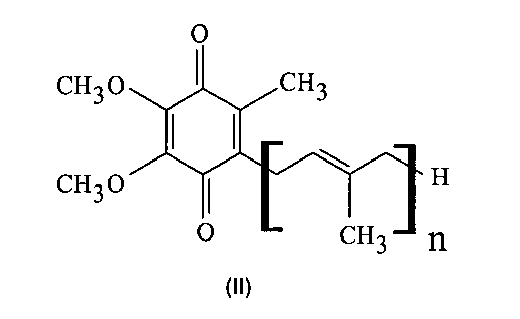 mit n = 6, 7, 8, 9 oder 10.
Besonders bevorzugt ist das Ubichinon der Formel (II) mit n = 10, auch bekannt als Coenzym Q10.
Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß besonders bevorzugte Zusammensetzungen sind dadurch gekennzeichnet, dass sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für N-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure und/oder Oleanol. Erfindungsgemäß besonders bevorzugte Zusammensetzungen sind dadurch gekennzeichnet, dass sie Oleanolsäure und/oder Oleanol in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®, Exsymol)
- ascorbylmethylsilanol pectinate (Ascorbosilane C®, Exsymol)
- dimethyl oxobenzodioxsilane (DSBC®, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsifanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C®, Exsymol).

Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Zusammensetzung.

In einer weiteren bevorzugten Ausführungsform enthalten die erfindungsgemäßen Zusammensetzungen Phytinsäure. Erfindungsgemäß besonders bevorzugte kosmetische oder dermatologische Zusammensetzungen sind dadurch gekennzeichnet, dass sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Zusammensetzung.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.

Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®} von Solabia mit der INCl-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft^{®} von Solabia mit der INCl-Bezeichnung Biosaccharide Gum-2. Ein erfindungsgemäß besonders bevorzugtes, Desoxyzucker-Bausteine enthaltendes Polysaccharid ist das Handelsprodukt Fucogenol^{®} von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm^{®} von Solabia mit der INCl-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia.

Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

Die erfindungsgemäßen kosmetischen oder dermatologischen topischen Zusammensetzungen, die mindestens ein Oligopeptid, das einen N-C₂-C₂₄-Acylrest aufweisen und/oder verestert sein kann, enthalten, sind hervorragend zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut geeignet.

Ein weiterer Gegenstand der vorliegenden Erfindung ist daher die Verwendung einer kosmetischen oder dermatologischen topischen Zusammensetzung, die mindestens ein Oligopeptid, das einen N-C₂-C₂₄-Acylrest aufweisen und/oder verestert sein kann, und mindestens einen Apfelextrakt, insbesondere Apfelkern- oder Apfelfruchtextrakt enthält, zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, zur Glättung der Haut und zur Verbesserung der Elastizität der Haut.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, zur Glättung der Haut und zur Verbesserung der Elastizität der Haut, das dadurch gekennzeichnet ist, dass eine kosmetische oder dermatologische topische Zusammensetzung, die mindestens ein Oligopeptid , das einen N-C₂-C₂₄-Acylrest aufweisen und/oder verestert sein kann, und mindestens einen Apfelextrakt, insbesondere Apfelkern- oder Apfelfruchtextrakt enthält, auf die Haut aufgetragen wird.

Bevorzugt liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels oder Oleogels, einer ein- oder mehrphasigen Lösung, eines Schaumes, eines Puders oder einer Mischung mit mindestens einem als medizinischer Klebstoff geeigneten Polymer vor. Die Zusammensetzungen können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonders bevorzugten Ausführungsform liegen die erfindungsgemäßen Zusammensetzungen als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den drei Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen. Die erfindungsgemäß verwendeten Wirkstoffe, insbesondere die Peptidwirkstoffe, werden bevorzugt bei allen Herstellverfahren, die ein Erhitzen erfordern, erst nach dem Erhitzen in die abgekühlte Formulierung eingearbeitet.
In der Ausführungsform als Emulsion oder als tensidisches Produkt, z. B. als Reinigungsmittel, enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospholipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäßen Zusammensetzungen enthalten die Emulgatoren bevorzugt in Mengen von 0,1 bis 25 Gew.-%, insbesondere 0,5-15 Gew.-%, bezogen auf die gesamte Zusammensetzung.

In einer weiteren besonders bevorzugten Ausführungsform ist mindestens ein nichtionischer Emulgator mit einem HLB-Wert von 8 und darunter enthalten. Derart geeignete Emulgatoren sind besonders bevorzugt Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4-6 C-Atomen und 2-5 Hydroxylgruppen ist. Besonders bevorzugte Emulgatoren sind solche, in denen R² Wasserstoff ist, insbesondere Behenylalkohol und Arachidylalkohol. Derartige Emulgatoren bilden sogenannte Lamellaremulsionen, deren Emulsionströpfchen von einer flüssig-kristallinen, lamellaren Phase aus Lipidmolekülen und Wasser umgeben und dadurch besonders stabilisiert und zur Wiederherstellung des gestörten Ordnungsgrades geschädigter Haut besonders geeignet sind. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

Besonders bevorzugte erfindungsgemäße Zusammensetzungen sind dadurch gekennzeichnet, dass mindestens ein nichtionischer Emulgator mit einem HLB-Wert von 8 und darunter mit der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2-5 Hydroxylgruppen ist, in Gesamtmengen von 0,5-10 Gew.-%, bevorzugt 1-6 Gew.-% und besonders bevorzugt 2-4 Gew.-%, enthalten ist.

Weitere geeignete Zusatzstoffe sind Fettstoffe, insbesondere pflanzliche Öle, wie Olivenöl, Sonnenblumenöl, Sojaöl, Rapsöl, Mandelöl, Jojobaöl, Orangenöl, Weizenkeimöl, Pfirsichkernöl und die flüssigen Anteile des Kokosöls, flüssige Paraffinöle, Isoparaffinöle und synthetische Kohlenwasserstoffe, Di-n-alkylether mit insgesamt 12 bis 36 C-Atomen, z. B. Di-n-octylether und n-Hexyl-n-octylether, Fettsäuren, besonders lineare und/oder verzweigte, gesättigte und/oder ungesättigte C₈₋₃₀-Fettsäuren, Fettalkohole, besonders gesättigte, ein- oder mehrfach ungesättigte, verzweigte oder unverzweigte Fettalkohole mit 6 - 30 Kohlenstoffatomen, Esteröle, das heißt Ester von C₆₋₃₀-Fettsäuren mit C₂₋₃₀-Fettalkoholen, Hydroxycarbonsäurealkylester, Dicarbonsäureester wie Di-n-butyladipat sowie Diolester wie Ethylenglykoldioleat oder Propylenglykoldi(2-ethylhexanoat), symmetrische, unsymmetrische oder cyclische Ester der Kohlensäure mit Fettalkoholen, z. B. Glycerincarbonat oder Dicaprylylcarbonat (Cetiol^{®} CC), Mono,- Di- und Trifettsäureester von gesättigten und/oder ungesättigten linearen und/oder verzweigten Fettsäuren mit Glycerin Wachse, insbesondere Insektenwachse, Pflanzenwachse, Fruchtwachse, Ozokerit, Mikrowachse, Ceresin, Paraffinwachse, Triglyceride gesättigter und gegebenenfalls hydroxylierter C₁₆₋₃₀-Fettsäuren, z. B. gehärtete Triglyceridfette, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane sowie Polydialkylsiloxane, die Amin- und/oder Hydroxy-Gruppen enthalten.

In einer bevorzugten Ausführungsform der Erfindung beträgt die Einsatzmenge der Fettstoffe 0,1 - 50 Gew.-%, bevorzugt 0,1 - 20 Gew.-% und besonders bevorzugt 0,1 - 15 Gew.-%, jeweils bezogen auf die gesamte Zusammensetzung.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Bevorzugte Handelsprodukte sind Sepigel^{®} 305, Simulgel^{®} 600, Simulgel^{®} NS, Simulgel^{®} EPG, Simulgel^{®} EG und Sepiplus^{®} 400 der Firma SEPPIC sowie Aristoflex^{®} AVC von Clariant. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Trübungsmittel und Perlglanzmittel wie Ethylenglykolmono- und -distearat und Treibmittel wie Propan-Butan-Gemische, N₂O, Dimethylether, CO₂ und Luft.

Die erfindungsgemäßen Zusammensetzungen können gegebenenfalls auch mindestens ein DNA-Reparaturenzym enthalten.

Die DNA-Reparaturenzyme können sowohl in Liposomen verkapselt als auch frei, das heißt unverkapselt, vorliegen. Die Liposomenverkapselung kann bevorzugt mit Phospholipiden, besonders bevorzugt mit Lecithin, erfolgen. Erfindungsgemäß besonders bevorzugt ist der Einsatz von mindestens einem liposomenverkapselten DNA-Reparaturenzym. Liposomenverkapselte Photolyase ist im Handel z. B. unter der Produktbezeichnung Photosomes^{™} (INCl-Bezeichnung: Aqua, Lecithin, Plankton Extract) liposomenverkapselte T4N5 z. B. unter der Bezeichnung Ultrasomes^{™} (INCl-Bezeichnung: Aqua, Lecithin, Micrococcus Lysate) von der Firma AGI Dermatics, USA, erhältlich. Da sich bei längerer Lagerung sowohl des Handelsproduktes als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor.

In den erfindungsgemäßen Zusammensetzungen sind die Handelsprodukte Photosomes^{™} oder Ultrasomes^{™} bevorzugt in Mengen von 0,1 - 10 Gew.-%, besonders bevorzugt 0,5 - 5,0 Gew.-% und außerordentlich bevorzugt 1,0 - 4,0 Gew.-%, bezogen auf das gesamte Mittel, enthalten. Bevorzugte erfindungsgemäße Zusammensetzungen sind dadurch gekennzeichnet, dass mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Insbesondere bevorzugt ist dabei das DNA-Reparaturenzym ausgewählt ist aus Photolyase und T 4 Endonuclease V sowie Mischungen dieser Enzyme.

Photolyase und T4 Endonuclease V, letztere im weiteren mit "T4N5" abgekürzt, sind im Stand der Technik bereits als sogenannte DNA-Reparatur-Enzyme bekannt. Unter DNA-Reparatur ist erfindungsgemäß die Spaltung bzw. Entfernung von UV-induzierten Pyrimidindimeren aus der DNA zu verstehen. "Pyrimidindimer" ist die im Stand der Technik gebräuchliche Bezeichnung für Dimere, die photochemisch, z. B. durch UV B-Strahlen, aus bestimmten Pyrimidinbasen der DNA gebildet werden. Pyrimidin selbst ist keine DNA-Base, dennoch wird im folgenden der Begriff "Pyrimidindimer" anstatt des korrekten Terminus "Pyrimidinbasen-Dimer" verwendet. Die Dimerisierung an der Pyrimidinbase Thymin erfolgt, indem benachbarte Thymin-Einheiten eines DNA-Stranges zu einer tricyclischen Verbindung dimerisieren. Das Dimerisierungsprodukt, eine cis-syn-Cyclobutandipyrimidin-Einheit, kann Fehler bei der Übertragung des genetischen Codes auslösen. Von der Bildung der Pyrimidindimeren sind vor allem die epidermalen Keratinozyten betroffen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Die Liposomenverkapselung von T4N5 wird von Ceccoli et al., J. Invest. Dermatol. 93, 190 - 194, 1989, beschrieben. Den Einsatz von liposomenverkapselter T4N5 bzw. Photolyase in kosmetischen Mitteln beschreiben Yarosh (US 5,190,762; WO 94/14419 A1) und Gilchrest et al. (WO 94/17781 A1). Burmeister et al. (EP 0 707 844 A2) offenbaren Zusammensetzungen, die liposomenverkapselte Kombinationen von DNA-Reparatur-Enzymen mit Tyrosin, Tyrosinderivaten, Vitaminen oder Provitaminen der Vitamin-Gruppen A, C und E, Glycoprotein-Komplexen von Kupfer, Zink oder Magnesium, Forskolin, cyclischem Adenosinmonophosphat (c-AMP), Bioflavonoiden oder Emulgatoren mit einem HLB-Wert von 10 - 14 enthalten, sowie Verfahren zur Herstellung von kosmetischen Bräunungsmitteln und Haarpflegeprodukten. In neueren Veröffentlichungen, insbesondere EP 1 153 600 A2, WO 02/49593 A2 und DE 203 16 269 U1, sind weitere kosmetische Zusammensetzungen mit DNA-Reparaturenzymen offenbart. Auf die Offenbarung der genannten Dokumente wird hiermit im vollen Umfang Bezug genommen.

Gemäß einer weiteren ganz besonders bevorzugten Ausführungsform ist in der Zusammensetzung kein DNA-Reparaturenzym enthalten.
Überraschenderweise wurde gefunden, dass die erfindungsgemäßen Zusammensetzungen auch ohne die einen Zusatz von DNA-Reparaturenzymen in der Zusammensetzung eine besonders gute Wirkung aufweisen.
Die erfindungsgemäß bevorzugten Zusammensetzungen, die mindestens ein hydrolysiertes Sojaprotein, insbesondere ein Sojaproteinhydrolysat mit einem mittleren Molekulargewicht im Bereich von 600 - 1000 Dalton, insbesondere bevorzugt 800 Dalton, enthalten, sind überraschenderweise in der Lage, die Expression eines hauteigenen DNA-Reparaturenzyms anzuregen.

Eine erfindungsgemäße Zusammensetzung, die keine DNA-Reparaturenzyme enthält, ist besonders wünschenswert, da Vorbehalte in der Öffentlichkeit gegen Enzyme in Kosmetika vor allem aufgrund eines möglichen Sensibilisierungs- bzw. Allergierisikos existieren. Ein weiterer Vorbehalt gegen Enzyme in Kosmetika besteht aus ethischen Gründen.

Weiterhin ist es durch eine Verwendung der erfindungsgemäßen Zusammensetzungen ohne DNA-Reparaturenzyme möglich, ein kostengünstigere Formulierung herzustellen und somit die Vorzüge der erfindungsgemäßen Lehre kostengünstiger, d.h. einem breiteren Publikum anzubieten.

Die nachfolgenden Beispiele sollen den Gegenstand der Erfindung erläutern, ohne ihn hierauf zu beschränken.

### 1. Oel-in-Wasser-Emulsionen

### 1. Beispielserie:

| | **1** | **2** | **3** | **4** | **5** | **6** | **7** |
|---|---|---|---|---|---|---|---|
| Lipoid S75-3 | 1,00 | 1,00 | 1,00 | 1,00 | 1,00 | 1,00 | 1,00 |
| Isopropylstearate | 4,00 | 4,00 | 4,00 | 4,00 | 4,00 | 4,00 | 4,00 |
| Cetiol B | 2,00 | 2,00 | 2,00 | 2,00 | 2,00 | 2,00 | 2,00 |
| Tocopherylacetat | 0,50 | 0,50 | 0,50 | 0,50 | 0,50 | 0,50 | 0,50 |
| Cutina MD | 1,00 | 1,00 | 1,00 | 1,00 | 1,00 | 1,00 | 1,00 |
| Stenol 1618 | 0,50 | 0,50 | 0,50 | 0,50 | 0,50 | 0,50 | 0,50 |
| Lanette 22 | 2,00 | 2,00 | 2,00 | 2,00 | 2,00 | 2,00 | 2,00 |
| Baysilone M 350 | 0,50 | 0,50 | 0,50 | 0,50 | 0,50 | 0,50 | 0,50 |
| Dow Corning 9040 | 1,00 | 1,00 | 1,00 | 1,00 | 1,00 | 1,00 | 1,00 |
| Glycerin | 4,50 | 4,50 | 4,50 | 4,50 | 4,50 | 4,50 | 4,50 |
| Hexandiol | 6,00 | 6,00 | 6,00 | 6,00 | 6,00 | 6,00 | 6,00 |
| Tego Carbomer 140 | 0,40 | 0,40 | 0,40 | 0,40 | 0,40 | 0,40 | 0,40 |
| Propylenglycol | 5,00 | 5,00 | 5,00 | 5,00 | 5,00 | 5,00 | 5,00 |
| Ederline L | 1,50 | 1,50 | 1,50 | 1,50 | 1,50 | 1,50 | 1,50 |
| Matrixyl 3000 | 2,00 | 2,00 | 2,00 | 2,00 | 2,00 | 2,00 | 2,00 |
| SYN®-Coll | 2,00 | - | - | - | - | - | - |
| Kombuchka | - | 3,00 | - | - | - | - | - |
| Ridulisse | - | - | 2,00 | - | - | - | - |
| Natrulon RC 50 DG | - | - | - | 3,00 | - | - | - |
| Glistin | - | - | - | - | 1,00 | - | - |
| Olea Europea | - | - | - | - | - | 0,10 | - |
| RetiSTAR | - | - | - | - | - | - | 0,50 |
| Simulgel NS | 1,50 | 1,50 | 1,50 | 1,50 | 1,50 | 1,50 | 1,50 |
| TiO2 | 0,50 | 0,50 | 0,50 | 0,50 | 0,50 | 0,50 | 0,50 |
| Phenoxyethanol | 0,40 | 0,40 | 0,40 | 0,40 | 0,40 | 0,40 | 0,40 |
| Methylparaben | 0,20 | 0,20 | 0,20 | 0,20 | 0,20 | 0,20 | 0,20 |
| Propylparaben | 0,20 | 0,20 | 0,20 | 0,20 | 0,20 | 0,20 | 0,20 |
| Parfum | 0,35 | 0,35 | 0,35 | 0,35 | 0,35 | 0,35 | 0,35 |
| Aqua | ad.100 | ad.100 | ad.100 | ad.100 | ad. 100 | ad.100 | ad.100 |

### 2. Beispielserie:

| | **1** | **2** | **3** | **4** | **5** | **6** | **7** |
|---|---|---|---|---|---|---|---|
| Montanov 68 | 5,00 | 5,00 | 5,00 | 5,00 | 5,00 | 5,00 | 5,00 |
| Myritol 316 | 8,00 | 8,00 | 8,00 | 8,00 | 8,00 | 8,00 | 8,00 |
| Cetiol SN | 2,00 | 2,00 | 2,00 | 2,00 | 2,00 | 2,00 | 2,00 |
| Saffloröl (Distelöl) | 2,00 | 2,00 | 2,00 | 2,00 | 2,00 | 2,00 | 2,00 |
| Stenol 1618 | 0,50 | 0,50 | 0,50 | 0,50 | 0,50 | 0,50 | 0,50 |
| Cutina MD | 1,00 | 1,00 | 1,00 | 1,00 | 1,00 | 1,00 | 1,00 |
| Lanette 22 | 0,50 | 0,50 | 0,50 | 0,50 | 0,50 | 0,50 | 0,50 |
| Cetiol SB 45 | 1,00 | 1,00 | 1,00 | 1,00 | 1,00 | 1,00 | 1,00 |
| Baysilon M 350 | 0,50 | 0,50 | 0,50 | 0,50 | 0,50 | 0,50 | 0,50 |
| Glycerin 86% | 5,00 | 5,00 | 5,00 | 5,00 | 5,00 | 5,00 | 5,00 |
| Hexandiol | 6,00 | 6,00 | 6,00 | 6,00 | 6,00 | 6,00 | 6,00 |
| Tego Carbomer 140 | 0,30 | 0,30 | 0,30 | 0,30 | 0,30 | 0,30 | 0,30 |
| Dry Flo Plus | 1,00 | 1,00 | 1,00 | 1,00 | 1,00 | 1,00 | 1,00 |
| Simulgel NS | 0,25 | 0,25 | 0,25 | 0,25 | 0,25 | 0,25 | 0,25 |
| Ederline L | 1,50 | 1,50 | 1,50 | 1,50 | 1,50 | 1,50 | 1,50 |
| Matrixyl 3000 | 2,00 | 2,00 | 2,00 | 2,00 | 2,00 | 2,00 | 2,00 |
| SYN®-Coll | 2,00 | - | - | - | - | - | - |
| Kombuchka | - | 3,00 | - | - | - | - | - |
| Ridulisse | - | - | 2,00 | - | - | - | - |
| Natrulon RC 50 DG | - | - | - | 3,00 | - | - | - |
| Glistin | - | - | - | - | 1,00 | - | - |
| Olea Europea | - | - | - | - | - | 0,10 | - |
| RetiSTAR | - | - | - | - | - | - | 0,50 |
| Parfum | 0,35 | 0,35 | 0,35 | 0,35 | 0,35 | 0,35 | 0,35 |
| Aqua | ad.100 | ad.100 | ad.100 | ad.100 | ad.100 | ad.100 | ad.100 |

### 3. Beispielserie:

| | **1** | **2** | **3** | **4** | **5** | **6** | **7** |
|---|---|---|---|---|---|---|---|
| Lipoid S75-3 | 0,50 | 0,50 | 0,50 | 0,50 | 0,50 | 0,50 | 0,50 |
| Isopropylstearate | 4,00 | 4,00 | 4,00 | 4,00 | 4,00 | 4,00 | 4,00 |
| Cetiol B | 2,00 | 2,00 | 2,00 | 2,00 | 2,00 | 2,00 | 2,00 |
| Tocopherylacetat | 0,50 | 0,50 | 0,50 | 0,50 | 0,50 | 0,50 | 0,50 |
| Cutina MD | 1,00 | 1,00 | 1,00 | 1,00 | 1,00 | 1,00 | 1,00 |
| Stenol 1618 | 0,50 | 0,50 | 0,50 | 0,50 | 0,50 | 0,50 | 0,50 |
| Lanette 22 | 2,00 | 2,00 | 2,00 | 2,00 | 2,00 | 2,00 | 2,00 |
| Baysilone M 350 | 0,50 | 0,50 | 0,50 | 0,50 | 0,50 | 0,50 | 0,50 |
| Dow Corning 9040 | 1,00 | 1,00 | 1,00 | 1,00 | 1,00 | 1,00 | 1,00 |
| Glycerin 86% | 4,50 | 4,50 | 4,50 | 4,50 | 4,50 | 4,50 | 4,50 |
| Hexandiol | 6,00 | 6,00 | 6,00 | 6,00 | 6,00 | 6,00 | 6,00 |
| Tego Carbomer 140 | 0,30 | 0,30 | 0,30 | 0,30 | 0,30 | 0,30 | 0,30 |
| DSH-CN | 5,00 | 5,00 | 5,00 | 5,00 | 5,00 | 5,00 | 5,00 |
| Propylenglycol | 5,00 | 5,00 | 5,00 | 5,00 | 5,00 | 5,00 | 5,00 |
| Ronaspheres LDP | 1,00 | 1,00 | 1,00 | 1,00 | 1,00 | 1,00 | 1,00 |
| Simulgel NS | 1,50 | 1,50 | 1,50 | 1,50 | 1,50 | 1,50 | 1,50 |
| Phenoxyethanol | 0,40 | 0,40 | 0,40 | 0,40 | 0,40 | 0,40 | 0,40 |
| Propylparaben | 0,20 | 0,20 | 0,20 | 0,20 | 0,20 | 0,20 | 0,20 |
| Methylparaben | 0,20 | 0,20 | 0,20 | 0,20 | 0,20 | 0,20 | 0,20 |
| Ederline L | 1,50 | 1,50 | 1,50 | 1,50 | 1,50 | 1,50 | 1,50 |
| Matrixyl 3000 | 2,00 | 2,00 | 2,00 | 2,00 | 2,00 | 2,00 | 2,00 |
| SYN®-Coll | 2,00 | - | - | - | - | - | - |
| Kombuchka | - | 3,00 | - | - | - | - | - |
| Ridulisse | - | - | 2,00 | - | - | - | - |
| Natrulon RC 50 DG | - | - | - | 3,00 | - | - | - |
| Glistin | - | - | - | - | 1,00 | - | - |
| Olea Europea | - | - | - | - | - | 0,10 | - |
| RetiSTAR | - | - | - | - | - | - | 0,50 |
| Parfum | 0,35 | 0,35 | 0,35 | 0,35 | 0,35 | 0,35 | 0,35 |
| Aqua | ad.100 | ad.100 | ad.100 | ad.100 | ad.100 | ad.100 | ad.100 |

### 2. Wasser-in-Siliconöl-Emulsion

| | **1** | **2** | **3** | **4** | **5** | **6** | **7** |
|---|---|---|---|---|---|---|---|
| Dow Corning 245 Fluid | 25,00 | 25,00 | 25,00 | 25,00 | 25,00 | 25,00 | 25,00 |
| Abil EM 90 | 2,00 | 2,00 | 2,00 | 2,00 | 2,00 | 2,00 | 2,00 |
| Hydrogenated Castor Oil | 2,00 | 2,00 | 2,00 | 2,00 | 2,00 | 2,00 | 2,00 |
| Wacker Belsil DM 100 | 2,50 | 2,50 | 2,50 | 2,50 | 2,50 | 2,50 | 2,50 |
| Natriumchlorid | 2,00 | 2,00 | 2,00 | 2,00 | 2,00 | 2,00 | 2,00 |
| Glycerin 86% | 10,00 | 10,00 | 10,00 | 10,00 | 10,00 | 10,00 | 10,00 |
| Symdiol 68 | 0,50 | 0,50 | 0,50 | 0,50 | 0,50 | 0,50 | 0,50 |
| SUNPMMA-S | 2,00 | 2,00 | 2,00 | 2,00 | 2,00 | 2,00 | 2,00 |
| Phenoxyethanol | 0,40 | 0,40 | 0,40 | 0,40 | 0,40 | 0,40 | 0,40 |
| Ederline L | 1,50 | 1,50 | 1,50 | 1,50 | 1,50 | 1,50 | 1,50 |
| Matrixyl 3000 | 2,00 | 2,00 | 2,00 | 2,00 | 2,00 | 2,00 | 2,00 |
| SYN®-Coll | 2,00 | - | - | - | - | - | - |
| Kombuchka | - | 3,00 | - | - | - | - | - |
| Ridulisse | - | - | 2,00 | - | - | - | - |
| Natrulon RC 50 DG | - | - | - | 3,00 | - | - | - |
| Glistin | - | - | - | - | 1,00 | - | - |
| Olea Europea | - | - | - | - | - | 0,10 | - |
| RetiSTAR | - | - | - | - | - | - | 0,50 |
| Parfum | 0,35 | 0,35 | 0,35 | 0,35 | 0,35 | 0,35 | 0,35 |
| Aqua | ad. 100 | ad.100 | ad.100 | ad.100 | ad.100 | ad. 100 | ad.100 |

Zur Tabelle: Alle Mengenangaben sind in Gew.-%.

### Liste der verwendeten Rohstoffe

| | |
|---|---|
| Abil EM 90 (Degussa) | Cetyl PEG/PPG-10/1 Dimethicone |
| Baysilone M 350 (Bayer) | DIMETHICONE |
| Belsil DM 100 (Wacker) | Polydimethylsiloxan |
| Biopeptide CL (Sederma) | Aqua, N-Palmitoyl-Gly-His-Lys |
| Cetiol B (Cognis) | Diisopropyladipat |
| Cetiol SB 45 (Cognis) | Butyrospermum Parkii (Linne) |
| Cetiol SN (Cognis) | CETEARYL ISONONANOATE |
| Controx KS (Cognis) | Tocopherol, Hydrogenated Palm Glycerides Citrate |
| Crodarom Chardonnay L (Croda) | Propylene Glycol, Water, Vitis Vinifera (Grape) Seed Extract |
| Cutina MD (Cognis) | Glyceryl Stearate |
| Dow Corning 245 Fluid | Cyclomethicone |
| Dow Corning 9040 | Cyclomethicone and Dimethicone Crosspolymer |
| Dry Flo Plus (National Starch) | Aluminium Starch Octenylsuccinate |
| DSH-C N (Exsymol) | Water, Dimethylsilanol-Hyaluronate |
| Ederline L (Vincience) | Hexyldecanol, Pyrus Malus (Apple) Fruit Extract (3 Gew.-% Aktiv-substanz) |
| Eyeliss (Sederma) | Water, Glycerin, Hesperidin Methyl Chalcone, Steareth-20, Dipeptide-2, PALMITOYL TETRAPEPTIDE-3 |
| Glistin (Exsymol) | Water, L-Glutamylaminoethyl-indol (0,9 - 1,1 Gew.-% Aktivsubstanz) |
| Kombuchka (syn. Kombucha, Sederma) | Water, Glycerine, Hydroxyethylcellulose, Saccharomyces/Xylinum/Black Tea Ferment |
| Lanette^{®} 22 (Cognis) | Behenyl Alcohol |
| Lipochroman-6 | Dimethylmethoxy Chromanol |
| Lipoid S75-3 (Lipoid GmbH) | Water, Hydrogenated Lecithin |
| Matrixyl (Sederma) | GLYCERIN, BUTYLENE GLYCOL, AQUA (WATER), CARBOMER, POLYSORBATE-20, PALMITOYL-PENTAPEPTIDE-3 |
| Matrixyl 3000 (Sederma) | GLYCERIN, AQUA (WATER), BUTYLENE GLYCOL, CARBOMER, POLYSORBATE-20,PALMITOYL OLIGOPEPTIDE, PALMITOYL TETRAPEPTIDE-3 |
| Montanov 68 (Seppic) | Cetearyl Alcohol, Cetearyl Glucoside |
| Myritol 316 (Cognis) | Caprylic/Capric Triglyceride |
| Natrulon RC | L-Carnitin (50 %), Water (29 %), Polyglycerine-10 (21 %) |
| NOVATA AB (Cognis) | Cocoglycerides |
| Olivenblatt Extrakt (Frutarom) | Olea europaea fol. extract, pulverförmig |
| Phytokine (Coletica) | HYDROLYZED SOY PROTEIN |
| RetiStar (BASF) | Caprylic/Capric Triglyceride, Sodium Ascorbate, Tocopherol, Retinol (5-6 Gew.-% Aktivsubstanz Retinol) |
| Ridulisse C (Silab) | HYDROLYZED SOY PROTEIN (6-7 Gew.-% Aktivsubstanz) |
| Ronasphere LDP | SILICA, Cl 77891 (TITANIUM DIOXIDE), Cl 77491 (IRON OXIDES) |
| Sepivinol R (Seppic) | Wine Extract |
| Simulgel NS (Seppic) | Hydroxyethyl Acrylate / Sodium Acryloyl Dimethyltaurate Copolymer, Squalane, Polysorbate 60 |
| Stenol 1618 (Cognis) | Cetearyl alcohol |
| SUNPMMA-S (Sunjin Chemical Company (Impag)) | METHYL METHACRYLATE CROSSPOLYMER |
| Symdiol 68 (Symrise) | 1,2-Hexanediol, Caprylyl Glycol |
| SynColl (Pentapharm) | Glycerine, Water, Palmitoyl-lysyl-valyl-lysine bistrifluoracetate salt (PALMITOYL-TRIPEPTIDE-3) |
| Tego Carbomer 140 | Carbomer |

### In vivo-Untersuchungen zur Verringerung der Faltentiefe von Hautfalten

Jeweils 42 Testpersonen (Frauen zwischen 40 - 70 Jahren) wurden mit einer der nachfolgend angegebenen Cremeformulierungen behandelt. Dabei wurden zweimal täglich über einen Zeitraum von 4 Wochen 2 mg der Cremeformulierung pro 1 cm² Hautareal im periorbitalen Bereich (um die Augenhöhlen herum) aufgetragen. Vor und nach dem Behandlungszeitraum wurde das Hautprofil berührungsfrei mittels der FOITS-Methode (Fast Optical in vivo Topometry of Human Skin) gemessen (die Methode ist beschrieben von M. Rohr, K. Schrader, SÖFW Journal 124, (1998), 52-59). Dabei erfolgte eine komplette dreidimensionale Erfassung des Hautoberflächenprofils mit anschließender statistischer Auswertung eventuell auftretender Änderungen des Profils anhand von DIN-Parametern. Die Differenz für die Rauigkeitsparameter Ra (Mittelrauwert) und Rz (gemittelte Rautiefe, siehe DIN 4768) vor und nach der Behandlung ist in der nachfolgenden Tabelle dargestellt.

Nach der oben beschriebenen Methode wurden Cremeformulierungen mit folgenden Wirkstoffkombinationen getestet:
- 2 % Matrixyl 3000 + 1,5 % Ederline L (E+M)
- 2 % Matrixyl 3000 + 1,5 % Ederline L + 2 % SynColl (E+M+S)
- 2 % Matrixyl 3000 + 1,5 % Ederline L + 3 % Natrulon RC (E+M+N)

Die Placebo-Cremeformulierung enthielt an Stelle der oben genannten Wirkstoffe Wasser in entsprechender Menge.

Ergebnisse der in vivo Anti-Falten-Studie mit erfindungsgemäßen Wirkstoffkombinationen

| Creme | Rz (µm) | Rz'(µm) Leerfeldkorrektur | Ra (µm) | Ra'(µm) Leerfeldkorrektur | Rz' (%) | Ra' (%) |
|---|---|---|---|---|---|---|
| Placebo | -10,2 | -7 | -2,1 | -1,5 | -5,30% | -5,40% |
| E+M | -12,7 | -9,5 | -2,3 | -1,7 | -7,10% | -6,30% |
| E+M+S | -12,7 | -9,5 | -2,3 | -1,7 | -7,10% | -6,20% |
| E+M+N | -15,8 | -12,6 | -2,8 | -2,2 | -9,30% | -7,80% |
| Leerfeld | -3,2 | 0 | -0,6 | 0 | | |

Es zeigte sich, dass die erfindungsgemäßen Wirkstoffkombinationen die gemittelte Rautiefe Rz' (inclusive Leerfeldkorrektur) gegenüber dem Placebo in überraschender Weise steigern konnten.

## Patentansprüche

1. Kosmetische oder dermatologische topische Zusammensetzung, enthaltend mindestens ein Oligopeptid, das mindestens einen N-C₂-C₂₄-Acylrest aufweisen und/oder verestert sein kann, und mindestens einen Apfelextrakt, insbesondere Apfelkern- oder Apfelfruchtextrakt, in einem geeigneten kosmetischen oder dermatologischen Träger.

2. Kosmetische oder dermatologische topische Zusammensetzung gemäß Anspruch 1, **dadurch gekennzeichnet, dass** sie zusätzlich mindestens einen weiteren Wirkstoff aufweist, der ausgewählt ist aus Retinol, Olivenblattextrakten, mindestens einem Proteinhydrolysat aus Sojabohnen, L-Carnitin und dessen Derivaten, sowie Kombuchka (synonym Kombucha), erhältlich aus der Fermentation von gezuckertem Schwarztee mit zwei symbiotischen Mikroorganismen Saccharomyces und Xylinum.

3. Kosmetische oder dermatologische topische Zusammensetzung gemäß einem der Ansprüche 1 oder 2, **dadurch gekennzeichnet, dass** das Oligopeptid ausgewählt ist aus Di-, Tri-, Tetra-, Penta- oder Hexapeptiden, die mit mindestens einem N-C₂-C₂₄-Acylrest acyliert und/oder verestert sein können, sowie Mischungen dieser Substanzen.

4. Kosmetische oder dermatologische topische Zusammensetzung gemäß einem der vorhergehenden Ansprüche, **dadurch gekennzeichnet, dass** das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus Gly-His-Lys und Gly-Gln-Pro-Arg, Kombinationen aus N-Palmitoyl-Gly-His-Lys und N-Palmitoyl-Gly-Gln-Pro-Arg, Kombinationen aus N-Palmitoyl-Gly-His-Lys und Gly-Gln-Pro-Arg, und Kombinationen aus Gly-His-Lys und N-Palmitoyl-Gly-Gln-Pro-Arg.

5. Kosmetische oder dermatologische topische Zusammensetzung gemäß einem der vorhergehenden Ansprüche, **dadurch gekennzeichnet, dass** sie mindestens eine der Oligopeptidkombinationen Gly-His-Lys und Gly-Gln-Pro-Arg, N-Palmitoyl-Gly-His-Lys und N-Palmitoyl-Gly-Gln-Pro-Arg, N-Palmitoyl-Gly-His-Lys und Gly-Gln-Pro-Arg, und Gly-His-Lys und N-Palmitoyl-Gly-Gln-Pro-Arg, enthält.

6. Kosmetische oder dermatologische topische Zusammensetzung gemäß Anspruch 5, **dadurch gekennzeichnet, dass** sie zusätzlich mindestens ein weiteres Oligopeptid enthält, das ausgewählt ist aus Lys-Val-Lys, dessen N-Acylderivaten, Estern und Salzen.

7. Kosmetische oder dermatologische topische Zusammensetzung gemäß einem der vorhergehenden Ansprüche, **dadurch gekennzeichnet, dass** sie zusätzlich mindestens ein L-Carnitin-Derivat, ausgewählt aus Acetyl-L-Carnitin, L-Carnitin-Fumarat, L-Carnitin-Citrat, Lauroyl-L-Carnitin und besonders bevorzugt L-Carnitin-Tartrat, enthält.

8. Kosmetische oder dermatologische topische Zusammensetzung gemäß einem der Ansprüche 2-7, **dadurch gekennzeichnet, dass** sie zusätzlich mindestens ein Sojaproteinhydrolysat mit einem mittleren Molekulargewicht im Bereich von 600 - 1000 Dalton, insbesondere bevorzugt 800 Dalton, enthält.

9. Kosmetische oder dermatologische topische Zusammensetzung gemäß einem der vorhergehenden Ansprüche, **dadurch gekennzeichnet, dass** der mindestens eine Apfelextrakt, insbesondere Apfelkernextrakt oder Apfelfruchtextrakt, in einer Gesamtmenge von 0,001 - 1 Gew.-%, besonders bevorzugt 0,01 - 0,5 Gew.-% und außerordentlich bevorzugt 0,03 - 0,1 Gew.-%, jeweils bezogen auf den Gehalt an aktiven Wirkstoffen in der gesamten topischen Zusammensetzung, enthalten ist.

10. Kosmetische oder dermatologische topische Zusammensetzung gemäß einem der vorhergehenden Ansprüche, **dadurch gekennzeichnet, dass** das mindestens eine Oligopeptid in Gesamtmengen von 0,000001 - 1 Gew.-%, bevorzugt 0,00001 - 0,1 Gew.-%, besonders bevorzugt 0,0001 - 0,001 - 0,01 - 0,05 Gew.-% und außerordentlich bevorzugt 0,002 - 0,005 Gew.-%, jeweils bezogen auf das Gewicht der kosmetischen oder dermatologischen topischen Zusammensetzung, enthalten ist.

11. Kosmetische oder dermatologische topische Zusammensetzung gemäß einem der vorhergehenden Ansprüche, **dadurch gekennzeichnet, dass** das Gewichtsverhältnis von Apfelextrakt zur Gesamtmenge des mindestens einen Oligopeptids, das mindestens einen N-C₂-C₂₄-Acylrest aufweisen und/oder verestert sein kann, im Bereich von 15 - 250, bevorzugt 20 - 200, besonders bevorzugt 30-175 und außerordentlich bevorzugt 50 bis 100 bis 150 liegt, wobei sich diese Angaben auf die jeweilige Aktivsubstanz beziehen.

12. Kosmetische oder dermatologische topische Zusammensetzung gemäß einem der vorhergehenden Ansprüche, **dadurch gekennzeichnet, dass** mindestens ein nichtionischer Emulgator mit einem HLB-Wert von 8 und darunter mit der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist, enthalten ist.

13. Kosmetische oder dermatologische topische Zusammensetzung gemäß einem der vorhergehenden Ansprüche, **dadurch gekennzeichnet, dass** mindestens ein Wirkstoff enthalten ist, der ausgewählt ist aus
a) natürlichen Betainverbindungen,
b) Vitaminen, Provitaminen und Vitaminvorstufen der Gruppen A, B, C, E, H und K und den Estern der vorgenannten Substanzen,
c) den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
d) Isoflavonoiden und Isoflavonoid-reichen Pflanzenextrakten,
e) Polyphenolen und Polyphenol-reichen Pflanzenextrakten,
f) Ubichinon und Ubichinol sowie deren Derivaten,
g) Silymarin,
h) natürlich vorkommenden Xanthin-Derivaten, ausgewählt aus Coffein, Theophyllin, Theobromin und Aminophyllin,
i) Ectoin,
j) Kreatin,
k) Olivenblattextrakten, Ursolsäure, Oleanol und/oder Oleanolsäure,
l) Mono- und Polyhydroxystilbenen und deren Estern,
m) Derivaten von methyliertem Silanol,
n) Phytinsäure,
o) Desoxyzuckern oder Desoxyzucker-Bausteine enthaltenden Polysacchariden.

14. Kosmetische oder dermatologische topische Zusammensetzung gemäß einem der vorhergehenden Ansprüche, **dadurch gekennzeichnet, dass** sie in einen Flockstift-Applikator abgefüllt ist.

15. Kosmetische oder dermatologische topische Zusammensetzung gemäß einem der vorhergehenden Ansprüche, **dadurch gekennzeichnet, dass** die Zusammensetzung kein DNA-Reparaturenzym enthält.

16. Verwendung einer kosmetischen oder dermatologischen topischen Zusammensetzung gemäß einem der Ansprüche 1 bis 15 zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, zur Glättung der Haut und zur Verbesserung der Hautelastizität.

17. Nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, zur Glättung der Haut und zur Verbesserung der Hautelastizität, das **dadurch gekennzeichnet ist, dass** eine kosmetische oder dermatologische topische Zusammensetzung gemäß einem der Ansprüche 1 bis 15 auf die Haut aufgetragen wird.
